# EUROPEAN PATENT SPECIFICATION

(11) **EP 3 968 019 B1**
(45) Date of publication and mention of the grant of the patent: **15.03.2023**
(21) Application number: 20213117.3
(22) Date of filing: 10.12.2020
(51) Int. Cl.: G01N 33/24, E21B 43/26, E21B 49/00

(54) **DEVICE FOR PHYSICAL SIMULATION OF FRACTURE NETWORK PROPAGATION BASED ON CO-EXPLOITATION OF THREE GASES OF UNCONVENTIONAL NATURAL GAS**
VORRICHTUNG ZUR PHYSIKALISCHEN SIMULATION VON BRUCHNETZWERKPROPAGATION BASIEREND AUF CO-AUSBEUTUNG VON DREI GASEN AUS UNKONVENTIONELLEM ERDGAS
DISPOSITIF DE SIMULATION PHYSIQUE DE LA PROPAGATION D'UN RÉSEAU DE FRACTURES SUR LA BASE DE LA CO-EXPLOITATION DE TROIS GAZ DE GAZ NATUREL NON CONVENTIONNEL

(30) Priority: 15.09.2020 CN 202010965046
(43) Date of publication of application: 16.03.2022
(73) Proprietor: Liaoning Technical University, Fuxin city, Liaoning (CN)
(72) Inventor: CHENG, Yao, Fuxin city, Liaoning (CN); LIN, Shan, Fuxin city, Liaoning (CN); MA, Yulin, Fuxin city, Liaoning (CN)
(74) Representative: Bjerkén Hynell KB

(56) References cited:
- CN-A- 104 060 976
- CN-A- 106 353 484
- CN-A- 109 540 688

## Description

### TECHNICAL FIELD

The invention relates to the technical field of coal mining experimental equipment, and in particular, to a device for physical simulation of fracture network propagation based on co-exploitation of three gases of unconventional natural gas.

### BACKGROUND

The three important unconventional natural gases, namely, coalbed methane, shale gas and tight sandstone gas ("the three gas" for short) features coexistence. In coal series stratum, coal seam and strata, shale layers and sandstone layers appear repeatedly and alternately, which can form multiple "source-reservoir-cap rock assemblages (SRCAs)", which is the key to the formation of various gas reservoirs. The source rocks in coal series stratum have a wide range of gas generation, various hydrocarbon gas geneses, great gas generation potential and a large amount of gas generated. Therefore, if only a single gas is exploited, the exploitation efficiency will be inevitably affected. Moreover, it is difficult to exploit a single gas, and there is also a low output and high cost. According to the experience of gas reservoir laminated mining, considering the coal series stratum as a whole, comprehensive exploration and development of shale gas and tight sandstone gas in coal measure coal seams can not only reduce exploration and development costs and increase total reserves of unconventional natural gas and technically recoverable resources, but also improve the use efficiency of gas wells, single well profits and production life. Therefore, "co-exploitation of three gases" can improve the economic benefits of unconventional natural gas exploitation, and is worth popularizing and applying.

A hydraulic fracturing technology is one of the core technologies of unconventional natural gas development. Different from methods for developing conventional oil and gas, the development of unconventional oil and gas reservoirs often needs to adopt large-scale hydraulic fracturing to transform reservoirs, pursue a complex fracturing network structure, and form a fracture network with natural fractures and artificial fractures interlaced with each other. This can break up effective reservoirs that can perform seepage, increase seepage area and flow conductivity, and improve initial production and ultimate recovery rate. Hydraulic fracturing leads to fracture network propagation. The generation of a fracture network by fracturing to improve reservoir permeability has been successfully applied in natural gas and shale gas industries.

Zhou Hao et al., taking Xujiahe Formation in West Sichuan Depression as an example, studied the reservoir forming mechanism, and believed that the combined exploitation of coalbed methane, shale gas and tight sandstone gas was feasible. Liang Bing et al., studied the reservoir forming mechanism, lithologic distribution characteristics, different gas-bearing reservoir characteristics, etc., and believed that three-gas exploitation of coal seam is feasible. However, because of interlayer interference, the output of a certain layer may be almost zero. The existing research shows that the interlayer interference in co-exploitation of three gases is mainly caused by the fracture network propagation. However, the existing research on co-exploitation of three gases mainly focuses on the reservoir forming mechanism and geological conditions, and does not involve co-exploitation of three gases. At present, many three-gas interference research devices or hydraulic fracturing physical simulation methods and devices are available, but none of them describe fracture network propagation and distribution of co-exploitation of three gases. Besides, none of them compare field experiments with physical simulation in a laboratory, and only perform single research, resulting in low accuracy of acquired data. Therefore, based on the core problem of three-gas co-exploitation, the invention provides a device and method for simulation of fracture network propagation based on hydraulic co-exploitation of three gases (shale gas, coalbed methane and tight gas) of unconventional natural gas reservoirs. The device and method are used to effectively simulate fracture network propagation and distribution based on co-exploitation of three gases, and combine field experiments with physical simulation in laboratories, thereby solving the above-mentioned problems of the prior art.

For example, patent application No. CN 104060976A belongs to the field of technical research on oil and gas reservoir development and particularly relates to a method for physically simulating sectional hydrofracture of different well types of perforated well shafts. The method comprises the following steps of: (1) processing a simulated shaft and arranging bullet holes on the well wall of the simulated shaft; (2) prefabricating the simulated shaft in the step (1) into an artificial core test sample and developing a hydrofracture physical simulation experiment by utilizing a true triaxial hydrofracture device system; and (3) observing fracture initiation and extension forms of hydraulic fractures at the positions of the bullet holes on the well wall of the simulated shaft by the hydrofracture physical simulation experiment in the step (2). According to the method, aiming at geological development blocks with different reservoir structures, different well types and fracture processes can be selected to carry out effective indoor simulation evaluation research; and the theoretical foundation is provided for design and scheme optimization of the hydrofracture process of conventional or unconventional dense oil and gas reservoirs and complex oil-gas blocked deposits.

Patent application No. CN 106353484A describes an experimental method and device for simulating composite multi-layer gas reservoir exploitation. The device comprises an injection system (1), a coal seam model system (2), a dense sandstone layer model system (3), a shale model system (4), a back pressure system (5), a recovery system (6) and a data acquisition system (7), wherein the injection system (1) is connected with the coal seam model system (2), the dense sandstone layer model system (3) and the shale model system (4) via a first multi-channel valve (81); the coal seam model system (2), the dense sandstone layer model system (3) and the shale model system (4) are connected with the back pressure system (5) via a second multi-channel valve (82); the back pressure system (5) is connected with the recovery system (6); and the data acquisition system (7) is connected with the coal seam model system (2), the dense sandstone layer model system (3), the shale model system (4) and the recovery system (6).

Patent application No. CN 109540688A describes a large-size true triaxial hydraulic fracturing simulation test device and a test method. The test device comprises a large-size true triaxial loading module, a hydraulic servopumping module, an acoustic emission positioning module, an infrared monitoring module, a sample loading and unloading module and a computer, wherein a loading plate is formed by combining an inner plate and an outer plate which are matched with each other through a spherical surface. The test method comprises the following steps of manufacturing and loading a test sample, and performing hydraulic fracturing simulation test, sample unloading, data processing and analysis and the like. The device and the method for a hydraulic fracturing simulation test of shale have the following advantages that the accuracy of a test result is not influenced by the sample processing size and the parallelism deviation, so that the requirements for the sample size processing precision are reduced, and the test reliability is improved; a loading condition of the shale in a triaxial loading chamber in a test process can be monitored in real time; the fracturing conditions of the multi-size samples can bemet; and a high-speed camera is matched for a uniaxial hydraulic fracturing simulation test, the dynamic expansion rule of hydraulic cracks can be observed directly, and multiple purposes of one machine can be realized.

### SUMMARY

The invention provides a device for physical simulation of fracture network propagation based on co-exploitation of three gases of unconventional natural gas. Through the device, real-time fracture propagation and cross-well interference experiments are carried out under different fracturing parameters and slickwater ratios, so as to fully describe the fracture network propagation law.

To achieve the above purpose, the invention provides the following technical solutions.

A device for physical simulation of fracture network propagation based on co-exploitation of three gases of unconventional natural gas as defined in the appended claims 1-6.

The invention achieves the following technical effects compared with the prior art:
According to the device for physical simulation of fracture network propagation based on co-exploitation of three gases of unconventional natural gas according to the invention, through hydraulic co-exploitation of shale gas, coalbed methane and tight gas, the visual observation of the evolution process of a three-gas co-exploitation reservoir and the interlayer interference are implemented together. A laboratory is connected with the field, and experimental data obtained by the two is comprehensively compared and analyzed to obtain simulation results closer to the actual field, thereby providing reliable technical data support for large-scale exploitation in the field. The invention solves the problem that the fracture network propagation and distribution and especially mutual interference occur in current three-gas co-exploitation. Besides, the invention combines field experiments with physical simulation in laboratories to achieve the purpose that obtained fracture propagation data of the fracture network is more accurate and has stronger field reference.

### BRIEF DESCRIPTION OF THE DRAWINGS

To describe the technical solutions in the examples of the invention or in the prior art more clearly, the following briefly introduces the accompanying drawings required for describing the examples. Apparently, the accompanying drawings in the following description show merely some examples of the invention and a person of ordinary skill in the art may still derive other accompanying drawings from these accompanying drawings without creative efforts.
FIG. 1 is a schematic diagram of the overall structure of a device for physical simulation of fracture network propagation based on co-exploitation of three gases of unconventional natural gas;
FIG. 2 is a schematic structural diagram a simulated rockshaft according to the invention; and
FIG. 3 is a schematic diagram showing the installation of acoustic emission probes according to the invention.

Reference numerals of the drawings: 1. sealed cabin; 2. main compartment; 3. simulated rockshaft; 4. gas reservoir horizontal well; 41. shale simulation layer; 42. coal simulation layer; 43. compact rock simulation layer; 5. acoustic emission positioning monitoring system; 51. acoustic emission signal amplifier; 52. full information acoustic emission recorder host; 6. loading mechanism; 7. acoustic emission probe; 8. high-speed camera; 9. transparent panel; 10. infrared camera; 11. base; 12, reinforced beam; 13. peripheral reinforcing strip; 14. infrared monitoring module; 15. infrared monitoring host.

### DETAILED DESCRIPTION

The following clearly and completely describes the technical solutions in the examples of the invention with reference to accompanying drawings in the examples of the invention.

The invention provides a device for physical simulation of fracture network propagation based on co-exploitation of three gases of unconventional natural gas. Through the device, real-time fracture propagation and cross-well interference experiments are carried out under different fracturing parameters and slickwater ratios so as to fully describe the fracture network propagation law.

In order to make the foregoing objectives, features, and advantages of the invention more understandable, the invention will be further described in detail below with reference to the accompanying drawings and specific implementations.

### Example 1

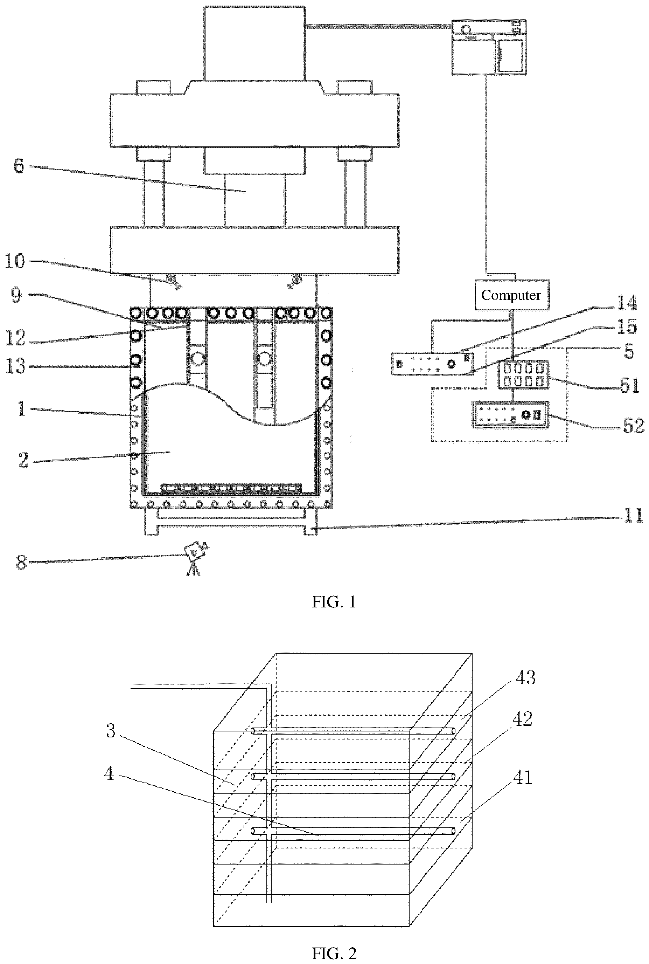

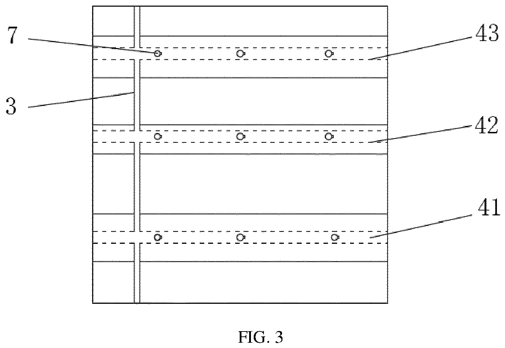

As shown in FIGs. 1-3, this example provides a device for physical simulation of fracture network propagation based on co-exploitation of three gases of unconventional natural gas. The device specifically includes: a sealed cabin 1 in which a main compartment 2 is formed; a simulated rockshaft 3 arranged in the main compartment 2, where three gas reservoir horizontal wells 4 are vertically arranged in the simulated rockshaft 3 and filled with a shale simulation layer, a coal simulation layer and a tight rock simulation layer from bottom to top respectively to simulate stratum distribution; each of the gas reservoir horizontal wells 4 is internally provided with a jet port configured to spray a fracturing fluid to implement hydraulic fracturing jet to a gas reservoir simulation layer; a loading mechanism 6 arranged at the top of the main compartment 2 and configured to apply pressure to the simulated rockshaft 3 to simulate stratum pressure; acoustic emission probes 7 arranged in the gas reservoir horizontal well 4 and configured to monitor the formation and propagation of a fracture network system in the gas reservoir simulation layer in real time during fracturing; and a high-speed camera 8 configured to visually record the evolution of the fracture network in each gas reservoir. Hydraulic fracturing of co-exploitation of three gases can be implemented through the simulated rockshaft 3 and the three gas reservoir horizontal wells therein. The simulated rockshaft 3 is preferably arranged on a middle portion of a transverse symmetry plane of the left of the main compartment 2, and the distance between the simulated rockshaft and the edge is determined according to actual geological conditions.

In this example, as shown in FIGs. 2-3, the three gas reservoir horizontal wells 4 are each provided with a valve to implement independent control of fracturing fluid injection in each horizontal well. During the experiment of each gas reservoir, the fracturing fluid enters the steering horizontal well through the single rockshaft to reach a target reservoir. By setting of the independent valves, it is convenient for operators to conduct a hydraulic fracturing experiment for a single gas reservoir or simultaneous hydraulic fracturing experiments for multiple gas reservoirs according to requirements, thereby expanding the application range of the device. Besides, during the simultaneous hydraulic fracturing experiments of the multiple gas reservoirs, because each gas reservoir is connected to the fracturing fluids through pipelines and the valves, thereby facilitating independent control of the types and injection pressures of the fracturing fluids in each gas reservoir. In this example, preferably, each gas reservoir is connected to a fracturing fluid source. When the simultaneous hydraulic fracturing experiments of the multiple gas reservoirs are carried out, the fracturing fluids can be injected into each layer simultaneously, thereby avoiding the error of experimental data caused by the time difference of fracturing fluid injection.

In this example, the jet ports are preferably bidirectional jet ports, and three bidirectional jet ports are arranged in each gas reservoir horizontal well at intervals. One acoustic emission probe 7 is arranged at a corresponding position of each jet port, and the acoustic emission probe 7 is externally connected to an acoustic emission positioning monitoring system 5 and configured to monitor the formation and propagation of a fracture network system in the gas reservoir simulation layer in real time during fracturing. The acoustic emission probe 7 preferably has a current acoustic emission probe structure with a positioning function and can detect a sound production position according to a fracture sound instantly made by fracture of the gas reservoir, track the continuous direction of the fracture sound, and can calculate a fracturing distance of the network fracture according to the speed of the detected sound wave. The acoustic emission positioning monitoring system 5 is internally provided with an acoustic emission signal amplifier 51 and a full information acoustic emission recorder host 52 which are electrically connected. The acoustic emission signal amplifier 51 is in signal connection with each acoustic emission probe 7. The full information acoustic emission recorder host 52 is connected to a computer and configured to report and save detection data of the acoustic emission probe 7 for data reserve of subsequent experimental analysis.

In this example, as shown in FIG. 1, a front end of the sealed cabin 1 is provided with a transparent panel 9, and the high-speed camera 8 is arranged outside the transparent panel 9. The high-speed camera 8 has a current camera structure and visually records the inversion of a high-definition displacement field, deformation field and velocity field by starting a DSCM mode and using the white-light speckle principle. The white-light speckle principle is well known in the art, and will not be repeated here.

In this example, as shown in FIG. 1, the top of the sealed cabin 1 is provided with an infrared camera 10 which is configured to perform experimental analysis related to fracture network propagation of gas reservoirs under the impact of infrared temperature and implement nondestructive monitoring of fracture network propagation in each gas reservoir.

In this example, as shown in FIG. 1, because it is necessary to implement large stress in a small range, the loading mechanism 6 is internally provided with a high-pressure press and a spherical loading plate, the high-pressure press is arranged on a top plate of the main compartment 2, and the axial pressure is over 30 MPa. The top and the bottom of the simulated rockshaft 3 are provided with a top plate and a bottom plate respectively. The spherical loading plate is installed at the bottom of the high-pressure press when the parallelism of a loading surface of the top plate of the simulated rockshaft is greater than 0.05 mm. This achieves the effect of auxiliary loading so as to ensure that the load is uniformly applied to the loading surface of the top plate of the simulated rockshaft and the pressure is stable.

Besides , this illustrative example not forming part of the invention provides a method for physical simulation of fracture network propagation based on co-exploitation of three gases of unconventional natural gas based on the foregoing device for physical simulation of fracture network propagation based on co-exploitation of three gases of unconventional natural gas. The method specifically includes the following steps.

Step 1: Drill three gas reservoir horizontal wells 4 in a simulated rockshaft 3 by means of the prior art, where the three gas reservoir horizontal wells are vertically distributed and filled with a shale simulation layer 41, a coal simulation layer 42 and a tight rock simulation layer 43 from bottom to top respectively. Each gas reservoir simulation layer is made in a similar material ratio. In this example, cement, lime, sand, water, coagulant, etc. are preferably used. The components of each gas reservoir simulation layer are basically the same. Due to different characteristics of different gas reservoirs, the ratios of the above components are different. The specific operation depends on the actual situation.

Step 2: Install the simulated rockshaft 3 filled with the gas reservoirs in a sealed cabin 1, and install one independent control valve on each of the gas reservoir horizontal wells 4 to perform independent control of fracturing fluid injection in each gas reservoir.

Step 3: Check the operation of an instrument and airtightness of the sealed cabin 1, connect each gas reservoir horizontal well 3 with a fracturing fluid through the valve, and start a loading mechanism 6 to slowly add load to a top plate of the simulated rockshaft 3 to a preset pressure.

Step 4: Inject the fracturing fluid into the three gas reservoir horizontal wells 4 simultaneously, start acoustic emission probes 7, a high-speed camera 8 and an infrared camera 10 simultaneously to record fracture network evolution during the fracturing process of each gas reservoir, and transmit acquired data to a computer for storage for later data analysis. The infrared camera 10 is electrically connected to the computer sequentially through an infrared monitoring module 14 and an infrared monitoring host 15. The high-speed camera 8 visually records the inversion of a white-light speckle high-definition displacement field, deformation field and velocity field in a DSCM mode. Through acoustic emission, DSCM and infrared monitoring, etc., the law of fracture network propagation is comprehensively described visually and indirectly, and the field is further combined with physical simulation in the laboratory to achieve the purpose that obtained fracture propagation data of the fracture network is more accurate and has stronger field reference.

Step 5: Replace one or more parameters of different fracturing fluids, different loading pressures or different fracturing fluid injection pressures, etc., and repeat step 4, so that hydraulic fracturing experiments under different parameters can be completed.

In this example, in step 4, the fracturing fluids introduced into each gas reservoir have different colors. The fracturing fluids in different colors represent the interaction and fracture network propagation process of different fractured reservoirs, which facilitates visual observation from the transparent panel 9.

In this example, the method further includes a verification step between step 2 and step 3, which adopts a current caliper to verify the surface parallelism of the loading surface of the top plate of the simulated rockshaft; when the surface parallelism is less than or equal to 0.05 mm, a uniform load is applied to the loading surface directly by a high-pressure press of the loading mechanism 6; when the surface parallelism is greater than 0.05 mm, a spherical loading plate is installed at the bottom of the high-pressure press to ensure that the load is uniformly applied to the loading surface of the top plate through the auxiliary action of the spherical loading plate. The verification principle and verification steps of surface parallelism mentioned above all pertain to the prior art, so they will not be described in detail here.

In this example, the transparent panel 9 is preferably an acrylic plate.

In this example, as shown in FIG. 1, two equally-spaced and vertically-parallel reinforced beams 12 are fixed in front of the sealed cabin 1; and peripheral reinforcing strips 19 are fixed around the sealed cabin 1 to increase the strength of the device. Besides, a bottom end of the sealed cabin 1 is also provided with a base 11.

It can be seen that the device and method for physical simulation of fracture network propagation based on co-exploitation of three gases of unconventional natural gas provided by this example are feasible to operate. The field and the laboratory are combined for analysis, and the simulation results are consistent with the actual hydraulic fracturing situation, which is used to study the fracture network propagation under the hydraulic fracturing conditions and provide reliable technical support for the actual hydraulic co-exploitation of three gases. Besides, The device and method for simulation of fracture network propagation based on hydraulic co-exploitation of three gases (shale gas, coalbed methane and tight gas) of unconventional natural gas reservoirs provided by this example solve the problems that the fracture network propagation and distribution and especially mutual interference occur in current three-gas co-exploitation and the field experiments are not compared with physical simulation in the laboratory.

The examples should be regarded as exemplary and non-limiting in every respect, and the scope of the disclosure is defined by the appended claims rather than the foregoing description. Any reference numeral in the claims should not be regarded as limiting the claims involved.
Specific examples are used for illustration of the principles and implementations of the invention.

The description of the foregoing examples is only used to help illustrate the method and its core ideas of the invention.

## Claims

1. A device for physical simulation of fracture network propagation based on co-exploitation of three gases of unconventional natural gas, comprising:
a sealed cabin in which a main compartment is formed, wherein a front end of the sealed cabin is provided with a transparent panel;
a simulated well arranged in the main compartment, wherein three gas reservoir horizontal wells are arranged along a vertical direction in the simulated well, and the three gas reservoir horizontal wells comprise a first gas reservoir horizontal well, a second gas reservoir horizontal well and a third gas reservoir horizontal well; the first gas reservoir horizontal well is filled with a shale simulation layer, the second gas reservoir horizontal well is filled with a coal simulation layer and the third gas reservoir horizontal well is filled with a tight rock simulation layer to simulate stratum distribution; the shale simulation layer, the coal simulation layer and the tight rock simulation layer are arranged from bottom to top, and the shale simulation layer, the coal simulation layer and the tight rock simulation layer extend along a horizontal direction; the first gas reservoir horizontal well runs along the shale simulation layer, the second gas reservoir horizontal well runs along the coal simulation layer, and the third gas reservoir horizontal well runs along the tight rock simulation layer; the first gas reservoir horizontal well is internally provided with first jet ports configured to spray a fracturing fluid flowing through a first pipeline into the first gas reservoir horizontal well to the shale simulation layer to implement hydraulic fracturing jet to the shale simulation layer, the second gas reservoir horizontal well is internally provided with second jet ports configured to spray a fracturing fluid flowing through a second pipeline into the second gas reservoir horizontal well to the coal simulation layer to implement hydraulic fracturing jet to the coal simulation layer, and the third gas reservoir horizontal well is internally provided with third jet ports configured to spray a fracturing fluid flowing through a third pipeline into the third gas reservoir horizontal well to the tight rock simulation layer to implement hydraulic fracturing jet to the tight rock simulation layer;
a loading mechanism arranged on a top of the main compartment and configured to apply pressure to a top plate arranged on a top of the simulated well to simulate stratum pressure;
acoustic emission probes arranged in each of the three gas reservoir horizontal wells and configured to monitor a formation and a propagation of a fracture network system in a respective one of the shale simulation layer, the coal simulation layer and the tight rock simulation layer in real time during fracturing; and
a high-speed camera arranged outside the transparent panel and configured to visually record evolution of the fracture network system in each of the shale simulation layer, the coal simulation layer and the tight rock simulation layer.

2. The device for physical simulation of fracture network propagation based on co-exploitation of three gases of unconventional natural gas according to claim 1, wherein the three gas reservoir horizontal wells are each provided with a valve to implement independent control of fracturing fluid injection in the first gas reservoir horizontal well, the second gas reservoir horizontal well and the third gas reservoir horizontal well.

3. The device for physical simulation of fracture network propagation based on co-exploitation of three gases of unconventional natural gas according to claim 1, wherein the first jet ports, the second jet ports and the third jet ports are bidirectional jet ports, and at least two of the bidirectional jet ports are arranged in each of the first gas reservoir horizontal well, the second gas reservoir horizontal well and the third gas reservoir horizontal well.

4. The device for physical simulation of fracture network propagation based on co-exploitation of three gases of unconventional natural gas according to claim 1, wherein a top of the sealed cabin is provided with an infrared camera.

5. The device for physical simulation of fracture network propagation based on co-exploitation of three gases of unconventional natural gas according to claim 1, wherein the loading mechanism comprises a high-pressure press and a spherical loading plate, wherein the high-pressure press is arranged at the top of the main compartment; and the spherical loading plate is installed at a bottom of the high-pressure press, and the spherical loading plate is configured to perform auxiliary loading on a loading surface of the top plate of the simulated well when parallelism of the loading surface of the simulated well is greater than 0.05 mm, so as to ensure that load is uniformly applied to the loading surface of the top plate of the simulated well.

6. The device for physical simulation of fracture network propagation based on co-exploitation of three gases of unconventional natural gas according to claim 1, further comprising an acoustic emission positioning monitoring system, wherein the acoustic emission positioning monitoring system is arranged outside the sealed cabin and configured to be in signal connection with the acoustic emission probe.

## Patentansprüche

1. Vorrichtung zur physikalischen Simulation von Bruchnetzwerkpropagation basierend auf Co-Ausbeutung von drei Gasen aus unkonventionellem Erdgas, umfassend:
eine abgedichtete Kabine, in der eine Hauptkammer ausgebildet ist, wobei ein vorderes Ende der abgedichteten Kabine mit einer transparenten Platte versehen ist;
ein simuliertes Bohrloch, das in der Hauptkammer angeordnet ist, wobei drei horizontale Gasreservoirbohrlöcher entlang einer vertikalen Richtung in dem simulierten Bohrloch angeordnet sind und die drei horizontalen Gasreservoirbohrlöcher ein erstes horizontales Gasreservoirbohrloch, ein zweites horizontales Gasreservoirbohrloch und ein drittes horizontales Gasreservoirbohrloch umfassen; wobei das erste horizontale Gasreservoirbohrloch mit einer Schiefersimulationsschicht gefüllt ist, das zweite horizontale Gasreservoirbohrloch mit einer Kohlesimulationsschicht gefüllt ist und das dritte horizontale Gasreservoirbohrloch mit einer dichten Gesteinssimulationsschicht gefüllt ist, um eine Schichtverteilung zu simulieren; wobei die Schiefersimulationsschicht, die Kohlesimulationsschicht und die dichte Gesteinssimulationsschicht von unten nach oben angeordnet sind und die Schiefersimulationsschicht, die Kohlesimulationsschicht und die dichte Gesteinssimulationsschicht sich entlang einer horizontalen Richtung erstrecken; wobei das erste horizontale Gasreservoirbohrloch entlang der Schiefersimulationsschicht verläuft, das zweite horizontale Gasreservoirbohrloch entlang der Kohlesimulationsschicht verläuft und das dritte horizontale Gasreservoirbohrloch entlang der dichten Gesteinssimulationsschicht verläuft; wobei das erste horizontale Gasreservoirbohrloch innen mit ersten Düsenöffnungen versehen ist, die dazu konfiguriert sind, ein Aufbrechfluid, das durch eine erste Rohrleitung in das erste horizontale Gasreservoirbohrloch fließt, auf die Schiefersimulationsschicht zu sprühen, um einen hydraulischen Aufbrechstrahl in die Schiefersimulationsschicht zu implementieren, das zweite horizontale Gasreservoirbohrloch innen mit zweiten Düsenöffnungen versehen ist, die dazu konfiguriert sind, ein Aufbrechfluid, das durch eine zweite Rohrleitung in das zweite horizontale Gasreservoirbohrloch fließt, auf die Kohlesimulationsschicht zu sprühen, um einen hydraulischen Aufbrechstrahl in die Kohlesimulationsschicht zu implementieren, und das dritte horizontale Gasreservoirbohrloch innen mit dritten Düsenöffnungen versehen ist, die dazu konfiguriert sind, ein Aufbrechfluid, das durch eine dritte Rohrleitung in das dritte horizontale Gasreservoirbohrloch fließt, auf die dichte Gesteinssimulationsschicht zu sprühen, um einen hydraulischen Aufbrechstrahl in die dichte Gesteinssimulationsschicht zu implementieren;
einen Belastungsmechanismus, der auf einer Oberseite der Hauptkammer angeordnet und dazu konfiguriert ist, Druck auf eine obere Platte auszuüben, die auf einer Oberseite des simulierten Bohrlochs angeordnet ist, um einen Schichtdruck zu simulieren;
Schallemissionssonden, die in jedem der drei horizontalen Gasreservoirbohrlöcher angeordnet und dazu konfiguriert sind, eine Bildung und Propagation eines Bruchnetzwerksystems jeweils in der Schiefersimulationsschicht, der Kohlesimulationsschicht und der dichten Gesteinssimulationsschicht in Echtzeit während des Aufbrechens zu überwachen; und
eine Hochgeschwindigkeitskamera, die außerhalb der transparenten Platte angeordnet und dazu konfiguriert ist, die Entwicklung des Bruchnetzwerks in jeder der Schiefersimulationsschicht, der Kohlesimulationsschicht und der dichten Gesteinssimulationsschicht visuell aufzuzeichnen.

2. Vorrichtung zur physikalischen Simulation von Bruchnetzwerkpropagation basierend auf Co-Ausbeutung von drei Gasen aus unkonventionellem Erdgas nach Anspruch 1, wobei die drei horizontalen Gasreservoirbohrlöcher jeweils mit einem Ventil versehen sind, um eine unabhängige Steuerung der Aufbrechfluideinspritzung in das erste horizontale Gasreservoirbohrloch, das zweite horizontale Gasreservoirbohrloch und das dritte horizontale Gasreservoirbohrloch umzusetzen.

3. Vorrichtung zur physikalischen Simulation von Bruchnetzwerkpropagation basierend auf Co-Ausbeutung von drei Gasen aus unkonventionellem Erdgas nach Anspruch 1, wobei die ersten Düsenöffnungen, die zweiten Düsenöffnungen und die dritten Düsenöffnungen bidirektionale Düsenöffnungen sind und mindestens zwei der bidirektionalen Düsenöffnungen in jedem des ersten horizontalen Gasreservoirbohrlochs, des zweiten horizontalen Gasreservoirbohrlochs und des dritten horizontalen Gasreservoirbohrlochs angeordnet sind.

4. Vorrichtung zur physikalischen Simulation von Bruchnetzwerkpropagation basierend auf Co-Ausbeutung von drei Gasen aus unkonventionellem Erdgas nach Anspruch 1, wobei eine Oberseite der abgedichteten Kabine mit einer Infrarotkamera versehen ist.

5. Vorrichtung zur physikalischen Simulation von Bruchnetzwerkpropagation basierend auf Co-Ausbeutung von drei Gasen aus unkonventionellem Erdgas nach Anspruch 1, wobei der Belastungsmechanismus eine Hochdruckpresse und eine sphärische Belastungsplatte umfasst, wobei die Hochdruckpresse an der Oberseite der Hauptkammer angeordnet ist; und die sphärische Belastungsplatte an einem Boden der Hochdruckpresse installiert ist und die sphärische Belastungsplatte dazu konfiguriert ist, eine Zusatzbelastung auf eine Belastungsfläche der oberen Platte des simulierten Bohrlochs auszuüben, wenn eine Parallelität der Belastungsfläche des simulierten Bohrlochs größer als 0,05 mm ist, um sicherzustellen, dass die Belastung gleichmäßig auf die Belastungsfläche der oberen Platte des simulierten Bohrlochs aufgebracht wird.

6. Vorrichtung zur physikalischen Simulation von Bruchnetzwerkpropagation basierend auf Co-Ausbeutung von drei Gasen aus unkonventionellem Erdgas nach Anspruch 1, ferner umfassend ein Schallemissionspositionsüberwachungssystem, wobei das Schallemissionspositionsüberwachungssystem außerhalb der abgedichteten Kabine angeordnet und dazu konfiguriert ist, in Signalverbindung mit der Schallemissionssonde zu stehen.

## Revendications

1. Dispositif de simulation physique de la propagation d'un réseau de fractures sur la base de la co-exploitation de trois gaz de gaz naturel non conventionnel, comprenant :
une cabine scellée dans laquelle un compartiment principal est formé, une extrémité avant de la cabine scellée étant pourvue d'un panneau transparent ;
un puits simulé agencé dans le compartiment principal, trois puits horizontaux de réservoir de gaz étant agencés le long d'une direction verticale dans le puits simulé, et les trois puits horizontaux de réservoir de gaz comprenant un premier puits horizontal de réservoir de gaz, un deuxième puits horizontal de réservoir de gaz et un troisième puits horizontal de réservoir de gaz ; le premier puits horizontal de réservoir de gaz est rempli d'une couche de simulation de schiste, le deuxième puits horizontal de réservoir de gaz est rempli d'une couche de simulation de charbon et le troisième puits horizontal de réservoir de gaz est rempli d'une couche de simulation de roche étanche pour simuler un agencement de strate ; la couche de simulation de schiste, la couche de simulation de charbon et la couche de simulation de roche étanche sont agencées de bas en haut, et la couche de simulation de schiste, la couche de simulation de charbon et la couche de simulation de roche étanche s'étendent le long d'une direction horizontale ; le premier puits horizontal de réservoir de gaz s'étend le long de la couche de simulation de schiste, le deuxième puits horizontal de réservoir de gaz s'étend le long de la couche de simulation de charbon, et le troisième puits horizontal de réservoir de gaz s'étend le long de la couche de simulation de roche étanche ; le premier puits horizontal de réservoir de gaz est pourvu intérieurement de premiers orifices de jet configurés pour pulvériser un fluide de fracturation s'écoulant à travers un premier pipeline dans le premier puits horizontal de réservoir de gaz vers la couche de simulation de schiste pour mettre en œuvre un jet de fracturation hydraulique vers la couche de simulation de schiste, le deuxième puits horizontal de réservoir de gaz est pourvu intérieurement de deuxièmes orifices de jet configurés pour pulvériser un fluide de fracturation s'écoulant à travers un deuxième pipeline dans le deuxième puits horizontal de réservoir de gaz vers la couche de simulation de charbon pour mettre en œuvre un jet de fracturation hydraulique vers la couche de simulation de charbon, et le troisième puits horizontal de réservoir de gaz est pourvu intérieurement de troisièmes orifices de jet configurés pour pulvériser un fluide de fracturation s'écoulant à travers un troisième pipeline dans le troisième puits horizontal de réservoir de gaz vers la couche de simulation de roche étanche pour mettre en œuvre un jet de fracturation hydraulique vers la couche de simulation de roche étanche ;
un mécanisme de chargement agencé sur un dessus du compartiment principal et configuré pour appliquer une pression à une plaque de dessus agencée sur un dessus du puits simulé pour simuler une pression de strate ;
des sondes d'émission acoustique agencées dans chacun des trois puits horizontaux de réservoir de gaz et configurées pour surveiller une formation et une propagation d'un système de réseau de fractures dans une couche respective de la couche de simulation de schiste, la couche de simulation de charbon et la couche de simulation de roche étanche en temps réel pendant la fracturation ; et
une caméra à grande vitesse agencée à l'extérieur du panneau transparent et configurée pour enregistrer visuellement l'évolution du système de réseau de fractures dans chaque couche parmi la couche de simulation de schiste, la couche de simulation de charbon et la couche de simulation de roche étanche.

2. Dispositif de simulation physique de la propagation d'un réseau de fractures sur la base de la co-exploitation de trois gaz de gaz naturel non conventionnel selon la revendication 1, dans lequel les trois puits horizontaux de réservoir de gaz sont chacun pourvus d'une soupape pour mettre en œuvre une régulation indépendante de l'injection de fluide de fracturation dans le premier puits horizontal de réservoir de gaz, le deuxième puits horizontal de réservoir de gaz et le troisième puits horizontal de réservoir de gaz.

3. Dispositif de simulation physique de la propagation d'un réseau de fractures sur la base de la co-exploitation de trois gaz de gaz naturel non conventionnel selon la revendication 1, dans lequel les premiers orifices de jet, les deuxièmes orifices de jet et les troisièmes orifices de jet sont des orifices de jet bidirectionnels, et au moins deux des orifices de jet bidirectionnels sont agencés dans chaque puits parmi le premier puits horizontal de réservoir de gaz, le deuxième puits horizontal de réservoir de gaz et le troisième puits horizontal de réservoir de gaz.

4. Dispositif de simulation physique de la propagation d'un réseau de fractures sur la base de la co-exploitation de trois gaz de gaz naturel non conventionnel selon la revendication 1, dans lequel un dessus de la cabine scellée est pourvu d'une caméra infrarouge.

5. Dispositif de simulation physique de la propagation d'un réseau de fractures sur la base de la co-exploitation de trois gaz de gaz naturel non conventionnel selon la revendication 1, dans lequel le mécanisme de chargement comprend une presse haute pression et une plaque de chargement sphérique, la presse haute pression étant agencée au niveau du dessus du compartiment principal ; et la plaque de chargement sphérique est installée au niveau d'un dessous de la presse à haute pression, et la plaque de chargement sphérique est configurée pour réaliser un chargement auxiliaire sur une surface de chargement de la plaque de dessus du puits simulé lorsque le parallélisme de la surface de chargement du puits simulé est supérieur à 0,05 mm, de façon à garantir que la charge soit appliquée uniformément à la surface de chargement de la plaque de dessus du puits simulé.

6. Dispositif de simulation physique de la propagation d'un réseau de fractures sur la base de la co-exploitation de trois gaz de gaz naturel non conventionnel selon la revendication 1, comprenant en outre un système de surveillance de positionnement d'émission acoustique, le système de surveillance de positionnement d'émission acoustique étant agencé à l'extérieur de la cabine scellée et configuré pour être en connexion de signal avec la sonde d'émission acoustique.
